Europäisches Patentamt

European Patent Office

Office européen des brevets

(11) Veröffentlichungsnummer: **0 544 097 A1**

# (12) EUROPÄISCHE PATENTANMELDUNG

(21) Anmeldenummer: **92117887.7**

(22) Anmeldetag: **20.10.92**

(51) Int. Cl.5: **C08L 67/04**, A61L 27/00,
A61L 25/00, A61K 9/20,
//(C08L67/04,67:04)

(30) Priorität: **23.10.91 DE 4134954**

(43) Veröffentlichungstag der Anmeldung:
**02.06.93 Patentblatt 93/22**

(84) Benannte Vertragsstaaten:
**AT BE CH DE DK ES FR GB GR IE IT LI LU NL
PT SE**

(71) Anmelder: **BOEHRINGER INGELHEIM KG**

**W-6507 Ingelheim am Rhein(DE)**

(84) **BE CH DE DK ES FR GR IE IT LI LU NL PT SE
AT**

(71) Anmelder: **BOEHRINGER INGELHEIM
INTERNATIONAL G.M.B.H.**

**W-6507 Ingelheim am Rhein(DE)**

(84) **GB**

(72) Erfinder: **Buchholz, Berthold, Dr.
Grundstrasse 55
W-6507 Ingelheim am Rhein(DE)**
Erfinder: **Entenmann, Günther,Dr. Dipl.-Chem.
Schützenpfad 16
W-6507 Ingelheim am Rhein(DE)**

(54) **Halbfeste Mischungen aus Oligomeren und/oder Polymeren auf der Basis von Milchsäure,
Verfahren zur deren Herstellung und deren Verwendung als resorbierbare Implantate.**

(57) Die vorliegende Erfindung betrifft halbfeste Mischungen aus Oligomeren und/oder Polymeren auf der Basis
von Milchsäure, deren Herstellung und deren Verwendung als resorbierbare Implantate.

EP 0 544 097 A1

Rank Xerox (UK) Business Services
(3.10/3.6/3.3.1)

Die vorliegende Erfindung betrifft halbfeste Mischungen aus Oligomeren und/oder Polymeren auf der Basis von Milchsäure, deren Herstellung und deren Verwendung als resorbierbare Implantate, wobei der Begriff Milchsäure im Sinne der vorliegenden Erfindung sowohl D- als auch L-Milchsäure umfaßt und die daraus aufgebauten Polymere bzw. Oligomere demzufolge D-, L- und D,L-Milchsäureeinheiten aufweisen können. - Derartige resorbierbare und körperverträgliche Materialien eignen sich u.a. als resorbierbare Implantate für die folgenden Anwendungen im menschlichen oder tierischen Organismus:

- lokale Blutstillung am eröffneten Knochen durch mechanische Tamponade (Knochenwachs)
- temporäre Ausfüllung von Gewebedefekten (Hart- und Weichgewebe)
- plastisches Matrixmaterial für die kontrollierte Freisetzung von Wirkstoffen (z.B. Antibiotika)

Physikalische Eigenschaften von resorbierbaren Polyestern auf der Basis von Milchsäure sind aus dem Stand der Technik bekannt [I. Engelberg und J. Kohn, Biomaterials 12 (1991) 292-304; A.U. Daniels et al., J. Appl. Biomat. 1 (1990) 57].

Hochmolekulare Polyester auf Basis von Milchsäure verkörpern unabhängig von ihrer Zusammensetzung bei Raum- oder Körpertemperatur Feststoffe ohne plastische Verformbarkeit. Je nach sterischer Einheitlichkeit können sie eine teilkristalline Morphologie aufweisen (Poly(L-lactid), Poly(D-lactid)) oder amorphe, glasartige Festkörper darstellen (Poly(D,L-lactid), Poly(meso-lactid)).

Im niedermolekularen Bereich zeigt sich eine starke Abhängigkeit der mechanischen und rheologischen Eigenschaften vom Molekulargewicht und von der Struktur:

Im Falle von Oligomeren der DL-Milchsäure, welche nicht kristallisationsfähig sind, ergibt sich mit wachsendem Molekulargewicht eine steigende Glasübergangstemperatur und bei Raumtemperatur ein gradueller Übergang von einer zähviskosen Flüssigkeit mit starker Fadenziehneigung zu glasartigen, spröden Feststoffen. Oligomere in einem Molekulargewichtsbereich von ca. 500 bis 2000 (Zahlenmittel) zeigen sowohl zähviskose als auch spröde Eigenschaften.

Bei Oligomeren der L- oder D-Milchsäure beeinflußt das Molekulargewicht die Kristallisationsfähigkeit und somit die Konsistenz. Bei einem Molekulargewicht unter ca. 400 sind die Oligomeren amorph. Ihre Eigenschaften entsprechen weitgehend denen der Oligo(DL-Milchsäuren). Oberhalb einem Molekulargewicht von ca. 700 kristallisieren die Kondensationsprodukte sehr leicht und bilden harte Festkörper. Im mittleren Molekulargewichtsbereich kann bei Raumtemperatur eine sehr langsame Kristallisation beobachtet werden, die durch Temperaturerhöhung beschleunigt werden kann. Die Konsistenz dieser Kondensate hängt somit sehr stark von der thermischen Vorbehandlung der Probe ab.

Aus dem Stand der Technik sind ebenfalls Vorschläge bekannt, durch Mischungen verschiedener resorbierbarer Polyester Materialien herzustellen, die gegenüber den Einzelkomponenten unterschiedliche Eigenschaftsprofile aufweisen.

So sind aus den US-PSen 47 19 246, 47 66 182, 48 00 219 und 49 81 696 Mischungen aus hochpolymerem Poly-(L-lactid) und Poly (D-lactid) bekannt. In derartigen Mischungen liegen Stereokomplexe mit einer hochschmelzenden kristallinen Phase und einer gegenüber den Einzelkomponenten verringerten Abbaugeschwindigkeit vor.

Die Europäische Patentanmeldung 401 844 offenbart Mischungen aus hochmolekrnlarem Poly(L-lactid) und Poly(D,L-lactid).

In der WO 90/01521 werden Mischungen aus Polylactiden beschrieben, die einen Zusatz an Lactid oder oligomeren Lactiden als Weichmacher enthalten.

Aus der US-PS 44 40 789 ist die Verwendung von Mischungen aus Polydioxanon und einer zweiten Komponente als resorbierbares Knochenwachs beschrieben. Als zweite Komponente finden Substanzen wie z.B. Sesamöl, Ricinusöl, Isopropylpalmitat, Polyethylenglycol sowie Ethylenglycol/Propylenglycol-Blockpolymere Verwendung.

In der kanadischen Offenlegungsschrift 12 60 488 werden Mischungen bestehend aus Copolymeren auf der Basis von Lactid sowie Glycolid und Ricinusöl vorgeschlagen.

Daneben ist aus dem Stand der Technik bekannt, daß niedermolekulare Copolymere von ε-Caprolacton und δ-Valerolacton eine wachsartige Konsistenz aufweisen und für eine Verwendung in Wirkstofffreigabesystemen geeignet sind [I. Imasaka et al., Int. J. Pharm. 68 (1991) 87].

Die US-PS 44 43 430 offenbart niedermolekulare Copolymere aus Lactid und Glycolid mit einem Lactidanteil zwischen 30 und 70 Mol-%, wobei das Molekulargewicht der Oligomeren in einem Bereich von 2000 und 2500 liegt.

Die EP 0 100 981 betrifft resorbierbare Wachse auf der Basis von Polyesteroligomeren sowie ein Verfahren zu deren Herstellung. Hierbei wird durch Zusatz von Alkoholen, Carbonsäuren oder Aminen während der Polymerisation bzw. Polykondensation das Molekulargewicht des resultierenden Polyesters geregelt und damit auf dessen Konsistenz Einfluß genommen.

Die Deutsche Offenlegungsschrift 37 16 302 hat die Verwendung von Polyester-Oligomeren von Glycol oder Milchsäure mit Glycerin als resorbierbare Knochenwachse zum Gegenstand.

Die Deutsche Offenlegungsschrift 38 25 211 offenbart Zusätze von organischen oder anorganischen Salzen zur Verbesserung der Konsistenzeigenschaften von Knochenwachsen, welche auf der Basis von Oligomeren der Glycol- oder Milchsäure hergestellt werden.

Schließlich beschreibt die Deutsche Offenlegungsschrift 38 26 915 Mischungen aus Keramikwerkstoffen und Polyester-Oligomeren der Milch- oder Glycolsäure mit ein- oder mehrfunktionellen Carbonsäuren oder Alkoholen als Knochenersatzmaterialien.

Die aus dem Stand der Technik bekannten Oligomeren der Milchsäure oder Glycolsäure weisen jedoch den Nachteil auf, daß sie - unabhängig vom Zusatz weiterer Komponenten wie z.B. Dodecanol, Ethylenglycol oder Glycerin - bei relativ niedrigem Molekulargewicht eine mehr oder weniger zäh-viskose Konsistenz besitzen, was mit einer ausgeprägten Neigung zum Fadenziehen und Eigenfließen verbunden ist. Beim Übergang zu höheren Molekulargewichten wird dagegen die Sprödigkeit der Oligomere erhöht.

Die Aufgabe der vorliegenden Erfindung besteht darin, ein resorbierbares und körperverträgliches Material für die eingangs genannten Anwendungen zur Verfügung zu stellen, welches die oben genannten Nachteile nicht besitzt und eine wachsartige bis pastöse Konsistenz aufweist.

Eine weitere Aufgabe der vorliegenden Erfindung besteht darin, ein Material zur Verfügung zu stellen, das bei Raum- bzw. Körpertemperatur - also in einem Temperaturbereich von ca. 20 bis ca. 40°C - plastisch verformbar ist.

Erfindungsgemäß werden die Aufgaben durch Mischungen von amorphen, zäh-viskosen Oligomeren auf der Basis von Milchsäure mit kristallinen Oligomeren oder Polymeren gelöst.

Erfindungsgemäß erfolgt die Herstellung der Blends durch einfaches Schmelzmischen, wobei die erforderliche Temperatur und Mischzeit von der Art, den Mengenanteilen und Molekulargewichten der eingesetzten Komponenten sowie von der Ansatzgröße abhängen. Um einen möglichen thermischen Molekulargewichtsabbau während des Mischens zu verhindern, ist es i.a. vorteilhaft, die Temperatur so niedrig wie möglich zu halten, so daß eine homogene Schmelze erhalten wird. Die in den Beispielen aufgeführten Mischungen zeigen, daß bei einer Temperatur von bis zu 160°C kein meßbarer Abbau eintritt. Die im Falle des Beispiels 12 durchgeführte GPC-Untersuchung zeigt weiterhin, daß eine Äquilibrierung der Molekulargewichte, beispielsweise durch Umesterungsreaktionen unter den vorliegenden Mischungsbedingungen nicht erfolgt.

Die für die Anwendung relevanten Eigenschaften der Mischungen, wie Konsistenz und Abbaugeschwindigkeit, können durch geeignete Wahl der Komponenten, der Zusammensetzung und der Molekulargewichte gesteuert werden.

Die in den Mischungen eingesetzten Oligomere oder Polymere können in bekannter Weise durch Polykondensation der entsprechenden Milchsäuren bzw. durch ringöffnende Polymerisation der entsprechenden Dilactone synthetisiert werden. Im Falle der Polykondensation ist hierbei der Zusatz eines Molekulargewichtsregulators nicht erforderlich, da der Oligomerisierungsgrad in einfacher Weise einzustellen ist, indem die Polykondensation beim gewünschten Entwässerungsgrad beendet wird.

Zur Herstellung der Mischungen aus Oligomeren und/oder Polymeren auf der Basis von Milchsäure geht man zweckmäßigerweise so vor, daß man die für die Mischung einzusetzenden Oligomere bzw. Polymere, die nach an sich bekannten Verfahren hergestellt wurden, mischt, bis zum Schmelzen erhitzt, die Schmelze homogenisiert und nach der Homogenisierung abkühlt und die halbfeste Mischung isoliert.

Für die Herstellung der Blends sind insbesondere die folgenden Polymere und Oligomere geeignet, wobei jede der kristallinen Komponenten mit jeder der amorphen Komponenten kombiniert werden kann:

Kristalline Oligomere oder Polymere:
- Oligo- und Poly(L-lactid)
- Oligo- und Poly(D-lactid)
- Blockcopolymere und Block-co-oligomere aus L- und D-Milchsäure
- Statistische Copolymere oder Oligomere, aus L- und D-Milchsäure, wobei zum Erhalt der Kristallinität der Anteil des jeweiligen Fremdmonomeren auf maximal ca. 10 % beschränkt ist.

Amorphe Oligomere:
- Oligo(L-lactat)
- Oligo(D-lactat)
- Oligo(DL-lactat)
- Oligo(meso-lactat)
- Statistische Cooligomere auf der Basis von D- und L-Milchsäure, sowie von D-, L-, DL- und meso-Lactid

- Cooligomere auf der Basis von Milchsäure mit einwertigen Alkoholen - wie z.B. Ethanol, Propanol, Isopropanol - oder mehrwertigen Alkoholen - wie z.B. Ethylenglykol, Propan-1,2-diol, Propan-1,3-diol, Glycerin, Erythrit, Sorbit, Mannit, Dulcit, - oder Kohlenhydrate - wie z.B. Fructose, Glucose, Maltose - oder Hydroxycarbonsäuren - wie z.B. Glycolsäure, $\beta$-Hydroxypropionsäure, $\alpha$-Hydroxyvaleriansäure, $\beta$-Hydroxyvaleriansäure, $\gamma$-Hydroxyvaleriansäure $\delta$-Hydroxyvaleriansäure - oder Polycarbonsäuren - wie z.B. Malonsäure, Bernsteinsäure, Glutarsäure, Adipinsäure - oder Hydroxycarbonsäuren - wie z.B. Zitronensäure, Weinsäure oder Äpfelsäure.

Aus der Vielzahl der möglichen Mischungskombinationen werden Mischungen aus einem niedermolekularen, zähflüssigen L-Lactid mit einem höhermolekularen, kristallinen L-Lactid bevorzugt, da in diesem Fall durch Hydrolyse lediglich L-Milchsäure, also eine körpereigene Substanz, generiert wird. Derartige Blends sind strukturell einheitlich und unterscheiden sich von konventionell hergestellten Oligomeren oder Polymeren der L-Milchsäure durch eine breitere und ggf. unsymmetrische Molekulargewichtsverteilung. Wie ein Vergleich der Beispiele 4 und 12 zeigt (beide Proben haben praktisch das gleiche mittlere Molekulargewicht), übt die Molekulargewichtsverteilung einen erheblichen Einfluß auf die Kristallisationsfähigkeit und damit auf die Konsistenz aus.

Wie schon eingangs erwähnt, sind für die Herstellung der Mischungen kristalline Polymere mit einem Molekulargewicht von mindestens 500 - zweckmäßigerweise 600 - geeignet. Als Polymere kommen sowohl statistische, als auch Homo- oder Blockpolymere in Frage.

Die vorliegende Erfindung betrifft somit Oligomer- oder Polymermischungen bestehend aus einem Homopolymeren der D- oder L-Milchsäure mit einem zahlenmittleren Molekulargewicht im Bereich von 600 bis 10000 und einem amorphen, zähviskosen Oligomeren auf der Basis von Milchsäure mit einem zahlenmittleren Molekulargewicht von kleiner als 500.

Die Erfindung betrifft insbesondere die oben genannten Oligomer- oder Polymermischungen in denen das Homopolymer ein zahlenmittleres Molekulargewicht im Bereich von 800 bis 8000 und das Oligomer auf der Basis von Milchsäure ein zahlenmittleres Molekulargewicht von kleiner 400 aufweist.

Die Erfindung betrifft vorzugsweise die oben genannten Oligomer- oder Polymermischungen, in denen das Homopolymer ein zahlenmittleres Molekulargewicht im Bereich von 1500 bis 6000 und das Oligomer auf der Basis von Milchsäure ein zahlenmittleres Molekulargewicht von kleiner 350 aufweist.

Die Erfindung betrifft besonders bevorzugt Oligomer- oder Polymermischungen, in denen das Homopolymer ein zahlenmittleres Molekulargewicht im Bereich von 1800 bis 4000 und das Oligomer auf der Basis von Milchsäure ein zahlenmittleres Molekulargewicht von kleiner 350 aufweist.

Die vorliegende Erfindung betrifft weiterhin Oligomer- oder Polymermischung bestehend aus einem Blockpolymeren der L- und D-Milchsäure mit einem zahlenmittleren Molekulargewicht im Bereich von 600 bis 10000 und einem amorphen zähviskosen Oligomeren auf der Basis von Milchsäure mit einem zahlenmittleren Molekulargewicht von kleiner 500.

Die Erfindung betrifft des weiteren insbesondere die oben genannten Oligomer- oder Polymermischungen in denen das Blockpolymer ein zahlenmittleres Molekulargewicht im Bereich von 800 bis 8000 und das Oligomer auf der Basis von Milchsäure ein zahlenmittleres Molekulargewicht von kleiner 400 aufweist.

Die Erfindung betrifft des weiteren vorzugsweise die oben genannten Oligomer- oder Polymermischungen in denen das Blockpolymer ein zahlenmittleres Molekulargewicht im Bereich von 1500 bis 6000 und das Oligomer auf der Basis von Milchsäure ein zahlenmittleres Molekulargewicht von kleiner 350 aufweist.

Die Erfindung betrifft des weiteren besonders bevorzugt Oligomer- oder Polymermischungen, in denen das Blockpolymer ein zahlenmittleres Molekulargewicht im Bereich von 1800 bis 4000 und das Oligomer auf der Basis von Milchsäure ein zahlenmittleres Molekulargewicht von kleiner 350 aufweist.

Die vorliegende Erfindung betrifft daneben Oligomer- oder Polymermischungen bestehend aus einem statistischem Copolymeren der L- und D-Milchsäure mit einem zahlenmittleren Molekulargewicht im Bereich von 600 bis 10000 und einem Gehalt an L-Milchsäureeinheiten zwischen 90 und 99 % oder 1 und 10 % sowie einem amorphen zähviskosen Oligomeren auf der Basis von Milchsäure mit einem Molekulargewicht von kleiner als 500.

Die Erfindung betrifft des weiteren insbesondere Oligomer- oder Polymermischungen in denen das statistische Polymer der L- und D-Milchsäure ein zahlenmittleres Molekulargewicht von im Bereich 800 bis 8000 und das Oligomer auf der Basis von Milchsäure ein Molekulargewicht von kleiner 400 aufweist.

Die Erfindung betrifft des weiteren vorzugweise Oligomer- oder Polymermischungen in denen das statistische Polymer der L- und D-Milchsäure ein zahlenmittleres Molekulargewicht im Bereich von 1500 bis 6000 und das Oligomer auf der Basis von Milchsäure ein Molekulargewicht von kleiner 350 aufweist.

Die Erfindung betrifft des weiteren besonders bevorzugt Oligomer- oder Polymermischungen, in denen das statistische Polymer ein zahlenmittleres Molekulargewicht im Bereich von 1800 bis 4000 und das Oligomer auf der Basis von Milchsäure ein zahlenmittleres Molekulargewicht von kleiner 350 aufweist.

Weiterhin hat die vorliegende Erfindung die oben beschriebenen Oligomer- oder Polymermischungen zum Gegenstand, wobei das Oligomere Einheiten der L-Milchsäure und/oder D-Milchsäure enthält.

Daneben betrifft die vorliegende Erfindung insbesondere Oligomer- oder Polymermischungen, in denen das Oligomere weitere Einheiten aus der Reihe der ein- oder mehrwertigen Alkohole aufweist und dieser Alkohol vorzugsweise durch Ethanol, Glycerin, Mannit, Sorbit, Ethandiol, 1.3-Propandiol oder 1.2-Propandiol verkörpert wird.

Des weiteren hat die vorliegende Erfindung insbesondere Oligomer- oder Polymermischungen zum Gegenstand, wobei das Oligomere weitere Einheiten aus der Reihe der Hydroxycarbonsäuren aufweist und diese Hydroxycarbonsäure vorzugsweise durch Glycolsäure Zitronensäure oder Weinsäure verkörpert wird.

Daneben betrifft die vorliegende Erfindung insbesondere Oligomer- oder Polymermischungen, wobei das Oligomere weitere Einheiten aus der Reihe der ein- oder mehrwertigen Carbonsäuren aufweist und diese Carbonsäure vorzugsweise durch Essigsäure oder eine zweiwertige Carbonsäure mit 3 bis 8 C-Atomen verkörpert wird.

Weiterhin hat die vorliegende Erfindung Oligomer- oder Polymermischungen zum Gegenstand, in denen das Oligomere weitere Einheiten aus der Reihe der Kohlenhydrate enthält und das Kohlenhydrat durch vorzugsweise Glucose verkörpert wird.

Die vorliegende Erfindung betrifft ferner Oligomer- oder Polymermischungen in denen der Anteil des zähviskosen Oligomeren insbesondere zwischen 5 und 95 Gewichtsprozent liegt.

Weiterhin hat die vorliegende Erfindung Oligomer- oder Polymermischungen zum Gegenstand, in denen der Anteil des zähviskosen Oligomeren vorzugsweise zwischen 30 und 70 Gew.-% liegt.

Daneben hat die vorliegende Erfindung ein Oligomer- oder Polymermischungen zum Gegenstand, in denen der Anteil des zähviskosen Oligomeren besonders bevorzugt ca. 50 % beträgt.

Daneben betrifft die vorliegende Erfindung Verfahren zu Herstellung von Mischungen aus Oligomeren und/oder Polymeren auf der Basis von Milchsäure, in dem man für die Mischung einzusetzenden Oligomere bzw. Polymere nach an sich bekannten Verfahren herstellt, mischt, bis zum Schmelzen erhitzt, die Schmelze homogenisiert und nach der Homogenisierung abkühlt und die halbfeste Mischung isoliert.

Weiterhin betrifft die vorliegende Erfindung die Verwendung der erfindungsgemäßen Oligomer- oder Polymermischungen als resorbierbares Implantat im menschlichen oder tierischen Organismus sowie zur temporären Ausfüllung von Hart- oder Weichgewebedefekten und insbesondere als resorbierbares Knochenwachs und als Matrixmaterial für die kontrollierte Freisetzung von Wirkstoffen.

Die eingangs genannten Aufgaben werden insbesondere durch die nachfolgend aufgeführten Beispiele gelöst. Verschiedenartige, andere und weitere Merkmale, Ausgestaltungen des Verfahrens und dergleichen, die der vorliegenden Erfindung zugeordnet sind, werden für den Fachmann aus der vorliegenden Beschreibung ersichtlich und in Verbindung mit den Beispielen, in welchen die gegenwärtig bevorzugten Ausführungsformen der Erfindung beispielhaft dargestellt sind, noch besser verständlich. - Es wird jedoch ausdrücklich darauf hingewiesen, daß die Beispiele und die diesen zugeordnete Beschreibung lediglich zum Zweck der Erläuterung und Beschreibung vorgesehen sind und nicht als Einschränkung der Erfindung anzusehen sind.

Beispiele

Vorbemerkungen:

Wenn nicht anders angegeben, bedeuten Molekulargewichtsangaben das zahlenmittlere Molekulargewicht ($M_N$), bestimmt durch Titration der Carboxylendgruppen. In den Tabellen ist das zahlenmittlere Molekulargewicht als tiefgestellter Index angegeben.

Beispiel 1
Oligo(L-lactat), $M_N$ = 315

Ein 4-1-Reaktionskolben mit Flügelrührer, Destillationsbrücke und Dephlegmator wird mit 3030 g L-Milchsäure (90 %) befüllt und unter Rühren auf ca. 20 Torr (26,664 mbar) evakuiert. Das Reaktionsgemisch wird auf 140°C aufgeheizt. Über einen Zeitraum von 5.5 Stunden wird das Lösungs- und Reaktionswasser abdestilliert. Während dieser Zeit wird die Temperatur bis auf 150°C erhöht. Nach der Reaktions wird die Oligomerschmelze ausgegossen und erkalten gelassen.

Das Kondensationsprodukt weist ein Molekulargewicht von 315 auf. Bei Raumtemperatur ist es hochviskos, fließfähig und transparent. Anzeichen einer Kristallisation sind nicht erkennbar.

Beispiel 2
Oligo(L-lactat), $M_N$ = 860

Die Herstellung erfolgt analog Beispiel 1, die Reaktionszeit beträgt jedoch 17 Stunden. Bereits während des Erkaltens setzt Kristallisation des Oligomeren ein. Das Molekulargewicht des Oligomeren beträgt 860. Es handelt sich um einen harten Feststoff mit einem Schmelzpunkt von 113°C (DSC, Heizrate 5K/min).

Beispiel 3
Oligo(D-lactat), $M_N$ = 345

Die Herstellung erfolgt analog Beispiel 1, abweichend hiervon wird jedoch 90 %ige D-Milchsäure eingesetzt. Das Molekulargewicht beträgt 345. Ebenso wie das Kondensationsprodukt aus Beispiel 1 handelt es sich um ein amorphes, hochviskoses Material.

Beispiel 4
Oligo(L-lactat), $M_N$ = 547 (Vergleichsbeispiel)

Aus dem Reaktionsansatz in Beispiel 1 wird nach einer Kondensationszeit von ca. 10 Stunden eine kleine Probe entnommen und analysiert. Das Molekulargewicht beträgt 547. Das Produkt ist bei Raumtemperatur transparent und glasartig hart, aber noch fließfähig. Auch nach 3 wöchiger Lagerung bei Raumtemperatur setzt keine Kristallisation ein.

Beispiele 5 bis 7
Mischungen aus Oligo(L-lactat) und Oligo(D-lactat)

Aus dem im Beispiel 3 beschriebenen Oligomeren aus D-Milchsäure und Oligomeren der L-Milchsäure mit einem Molekulargewicht von 1815 werden Mischungen hergestellt. Hierfür werden die jeweiligen Komponenten in einen Glaskolben eingewogen und anschließend über einen Zeitraum von 15 bis 30 Minuten unter Rührung in der Schmelze homogenisiert. Die Mischungstemperatur beträgt 160°C. Nach Homogenisierung wird die Schmelze aus dem Kolben ausgegossen und erkalten gelassen. Alle Mischungen der Beispiele 5 bis 8 kristallisieren beim Erkalten und werden intransparent.

| Zusammensetzung* | | $M_N$ | Konsistenz | Beispiel |
|---|---|---|---|---|
| $L_{1815}/D_{345}$ | | | | |
| 15 | 85 | 409 | wachsartig | 5 |
| 25 | 75 | 432 | wachsartig | 6 |
| 30 | 70 | 478 | wachsartig, leicht spröde | 7 |

* L bzw. D bedeutet ein Oligo- bzw. Poly-L- bzw. Poly-D-lactid (-lactat).

Die Mischung des Beispiels 7 weist einen Schmelzpunkt von 148°C auf (DSC, Heizrate 5 K/min).

Beispiele 8 bis 17
Mischungen aus verschiedenen Oligo(L-lactiden)

Aus Oligomeren oder Polymeren der L-Michsäure mit verschiedenen Molekulargewichten werden Mischungen hergestellt. Die Versuchsdurchführung erfolgt analog den Beispielen 5-7. Die Mischungstemperatur beträgt 140°C. Alle aufgeführten Mischungen kristallisieren beim Erkalten und werden intransparent.

| Zusammensetzung | | $M_N$ | Konsistenz | Beispiel |
|---|---|---|---|---|
| $L_{1815}/L_{315}$ | | | | |
| 10 | 90 | 348 | weich, zieht Fäden | 8 |
| 20 | 80 | 396 | wachsartig | 9 |
| 30 | 70 | 427 | wachsartig | 10 |
| 40 | 60 | 465 | wachsartig | 11 |
| 50 | 50 | 538 | wachsartig | 12 |
| 60 | 40 | 618 | wachsartig, leicht spröde | 13 |
| $L_{860}/L_{315}$ | | | | |
| 50 | 50 | 451 | weich, zieht Fäden | 14 |
| 70 | 30 | 557 | wachsartig | 15 |
| 80 | 20 | n.b.* | wachsartig | 16 |
| 90 | 10 | n.b.* | wachsartig, spröde | 17 |

\* n.b. = nicht bestimmt

Die Mischung des Beispiels 12 weist einen Schmelzpunkt von 125 °C auf (DSC, Heizrate 5 K/min).

Beispiel 18 GPC-Untersuchung an Mischung 12 An der Mischung des Beispiels 12 wurde exemplarisch eine GPC-Analyse durchgeführt, um zu untersuchen, ob es während der Schmelzmischung zu einer (teilweisen) Äquilibrierung der Molekulargewichte kommt. Als Vergleich hierzu wurde eine 1:1 - Mischung beider Ausgangskomponenten - hergestellt in Lösung - verwendet. Die GPC-Bestimmung wurde nach folgender Methode durchgeführt:

| | |
|---|---|
| Lösungsmittel: | Chloroform 0.5 mg pro ml |
| Injektionsvolumen: | 100 $\mu$l |
| Temperatur: | Raumtemperatur |
| Standard: | engverteilte Polystyrolstandards |
| Detektor: | RI-Detektor |
| Fluß: | 1 ml/min |
| Säulen: | PL-GEL Säulen 250/300/300 mm in den Ausschlußgrenzen 100, 1000, 100000 A. |
| Mischung 12: | $M_W$ = 5690, $M_N$ = 2300, $M_W/M_N$ = 2.5 |
| Vergleich: | $M_W$ = 5630, $M_N$ = 2280, $M_W/M_N$ = 2.5 |

**Patentansprüche**

1. Halbfeste, im Temperaturbereich von 20 bis 40°C plastisch verformbare Oligomer- oder Polymermischung bestehend aus einem Homopolymeren der D- oder L-Milchsäure mit einem zahlenmittleren Molekulargewicht im Bereich von 600 bis 10000 und einem amorphen, zähviskosen Oligomeren auf der Basis von Milchsäure mit einem zahlenmittleren Molekulargewicht von kleiner 500.

2. Oligomer- oder Polymermischung nach Anspruch 1, dadurch gekennzeichnet, daß das Homopolymer ein zahlenmittleres Molekulargewicht im Bereich von 800 bis 8000 und das Oligomer auf der Basis von Milchsäure ein zahlenmittleres Molekulargewicht von kleiner 400 aufweist.

3. Oligomer- oder Polymermischung nach Anspruch 1, dadurch gekennzeichnet, daß das Homopolymer ein zahlenmittleres Molekulargewicht im Bereich von 1500 bis 6000 und das Oligomer auf der Basis von Milchsäure ein zahlenmittleres Molekulargewicht von kleiner 350 aufweist.

4. Oligomer- oder Polymermischung nach Anspruch 1, dadurch gekennzeichnet, daß das Homopolymer ein zahlenmittleres Molekulargewicht im Bereich von 1800 bis 4000 und einem amorphen Oligomeren auf der Basis von Milchsäure mit einem zahlenmittleren Molekulargewicht kleiner 350 aufweist.

5. Halbfeste, in einem Temperaturbereich von 20 bis 40°C plastisch verformbare Oligomer- oder Polymermischung bestehend aus einem Blockpolymeren der L- und D-Milchsäure mit einem zahlenmittleren Molekulargewicht im Bereich von 600 bis 10000 und einem amorphen zähviskosen Oligomeren auf der Basis von Milchsäure mit einem zahlenmittleren Molekulargewicht von kleiner 500.

6. Oligomer- oder Polymermischung nach Anspruch 5, dadurch gekennzeichnet, daß das Blockpolymer ein zahlenmittleres Molekulargewicht im Bereich von 800 bis 8000 und das Oligomer auf der Basis von Milchsäure ein zahlenmittleres Molekulargewicht von kleiner 400 aufweist.

7. Oligomer- oder Polymermischung nach Anspruch 5, dadurch gekennzeichnet, daß das Blockpolymer ein zahlenmittleres Molekulargewicht im Bereich von 1500 bis 6000 und das Oligomer auf der Basis von Milchsäure ein zahlenmittleres Molekulargewicht von kleiner 350 aufweist.

8. Oligomer- oder Polymermischungen nach Anspruch 5, dadurch gekennzeichnet, daß das Blockpolymer ein zahlenmittleres Molekulargewicht im Bereich von 1800 bis 4000 und einem amorphen zähviskosen Oligomeren auf der Basis von Milchsäure mit einem zahlenmittleren Molekulargewicht kleiner 350 aufweist.

9. Halbfeste in einem Temperaturbereich von 20 bis 40°C plastisch verformbare Oligomer- oder Polymermischung bestehend aus einem statistischen Copolymeren der L- und D-Milchsäure mit einem zahlenmittleren Molekulargewicht im Bereich von 600 bis 10000 und einem Gehalt an L-Milchsäureeinheiten zwischen 90 und 99 % oder 1 und 10 % sowie einem amorphen zähviskosen Oligomeren auf der Basis von Milchsäure mit einem zahlenmittleren Molekulargewicht von kleiner 500.

10. Oligomer- oder Polymermischung nach Anspruch 9, dadurch gekennzeichnet, daß das statistische Polymer der L- und D-Milchsäure ein zahlenmittleres Molekulargewicht im Bereich von 800 bis 8000 und das Oligomer auf der Basis von Milchsäure ein Molekulargewicht von kleiner 400 aufweist.

11. Oligomer- oder Polymermischung nach Anspruch 9, dadurch gekennzeichnet, daß das statistische Polymer der L- und D-Milchsäure ein zahlenmittleres Molekulargewicht im Bereich von 1500 bis 6000 und das Oligomer auf der Basis von Milchsäure ein Molekulargewicht von kleiner 350 aufweist.

12. Oligomer- oder Polymermischung nach Anspruch 9, dadurch gekennzeichnet, daß das statistische Polymer ein zahlenmittleres Molekulargewicht im Bereich von 1800 bis 4000 und einem amorphen zähviskosen Oligomeren auf der Basis von Milchsäure mit einem zahlenmittleren Molekulargewicht kleiner 350 aufweist.

13. Mischung nach Anspruch 1 bis 12, dadurch gekennzeichnet, daß das Oligomere Einheiten der L-Milchsäure und/oder D-Milchsäure enthält.

**14.** Mischung nach einem der Ansprüche 1 bis 13, dadurch gekennzeichnet, daß das Oligomere weitere Einheiten aus der Reihe ein- oder mehrwertiger Alkohole aufweist.

**15.** Mischung nach Anspruch 14, dadurch gekennzeichnet, daß der Alkohol Ethanol ist.

**16.** Mischung nach Anspruch 14, dadurch gekennzeichnet, daß der Alkohol Glycerin ist.

**17.** Mischung nach Anspruch 14, dadurch gekennzeichnet, daß der Alkohol Ethandiol, 1.3-Propandiol oder 1.2-Propandiol ist.

**18.** Mischung nach Anspruch 14, dadurch gekennzeichnet, daß der Alkohol Mannit oder Sorbit ist.

**19.** Mischung nach einem der Ansprüche 1 bis 13, dadurch gekennzeichnet, daß das Oligomere weitere Einheiten aus der Reihe der Hydroxycarbonsäuren aufweist.

**20.** Mischung nach Anspruch 19, dadurch gekennzeichnet, daß die Hydroxycarbonsäure Glycolsäure ist.

**21.** Mischung nach Anspruch 19, dadurch gekennzeichnet, daß die Hydroxycarbonsäure Zitronensäure ist.

**22.** Mischung nach Anspruch 19, dadurch gekennzeichnet, daß die Hydroxycarbonsäure Weinsäure ist.

**23.** Mischung nach einem der Ansprüche 1 bis 13, dadurch gekennzeichnet, daß das Oligomere weitere Einheiten aus der Reihe ein- oder mehrwertiger Carbonsäuren aufweist.

**24.** Mischung nach Anspruch 23, dadurch gekennzeichnet, daß die Carbonsäure Essigsäure ist.

**25.** Mischung nach Anspruch 23, dadurch gekennzeichnet, daß die Carbonsäure eine zweiwertige Carbonsäure mit 3 bis 8 C-Atomen ist.

**26.** Mischung nach einem der Ansprüche 1 bis 13, dadurch gekennzeichnet, daß das Oligomere weitere Einheiten aus der Reihe der Kohlenhydrate enthält.

**27.** Mischung nach Anspruch 26, dadurch gekennzeichnet, daß das Kohlenhydrat Glucose ist.

**28.** Mischung nach einem der Ansprüche 1 bis 27, dadurch gekennzeichnet, daß der Anteil des zähviskosen Oligomeren zwischen 5 und 95 Gewichtsprozent liegt.

**29.** Mischung nach einem der Ansprüche 1 bis 28, dadurch gekennzeichnet, daß der Anteil des zähviskosen Oligomeren zwischen 30 und 70 Gew.-% liegt.

**30.** Mischung nach einem der Ansprüche 1 bis 28, dadurch gekennzeichnet, daß der Anteil des zähviskosen Oligomeren 50 % beträgt.

**31.** Verfahren zur Herstellung von Mischungen aus Oligomeren und/oder Polymeren auf der Basis von Milchsäure, dadurch gekennzeichnet, daß man die für die Mischungen einzusetzenden Oligomere bzw. Polymere nach an sich bekannten Verfahren herstellt, mischt, bis zum Schmelzen erhitzt, die Schmelze homogenisiert und nach der Homogenisierung abkühlt und die erkaltete Mischung isoliert.

**32.** Verwendung einer Mischung nach Anspruch 1 bis 30 als resorbierbares Implantat im menschlichen oder tierischen Organismus.

**33.** Verwendung einer Mischung nach Anspruch 1 bis 30 zur temporären Ausfüllung von Hart- oder Weichgewebedefekten.

**34.** Verwendung einer Mischung nach Anspruch 1 bis 30 als resorbierbares Knochenwachs.

**35.** Verwendung einer Mischung nach Anspruch 1 bis 30 als Matrixmaterial für die kontrollierte Freisetzung von Wirkstoffen.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 281 482 (RESEARCH TRIANGLE INSTITUTE) <br> * Ansprüche 1-13 * <br> --- | 1-13,35 | C08L67/04 <br> A61L27/00 <br> A61L25/00 <br> A61K9/20 |
| D,A | EP-A-0 100 981 (HENKEL KOMMANDITGESELLSCHAFT AUF AKTIEN) <br><br> * Ansprüche 1-7 * <br> --- | 1,14,15, 17,23, 24,32,34 | //C08L67/04, 67:04) |
| A <br><br> D | EP-A-0 352 588 (HENKEL KOMMANDITGESELLSCHAFT AUF AKTIEN) <br> * Ansprüche 1-10 * <br> & DE-A-3 825 211 <br> --- | 1-35 | |
| A <br><br> D | EP-A-0 290 983 (HENKEL KOMMANDITGESELLSCHAFT AUF AKTIEN) <br> * Ansprüche 1-5 * <br> & DE-A-3 716 302 <br> --- | 1,14,16, 32-34 | |
| D,A | WO-A-9 001 521 (BATELLE MEMORIAL INTSTITUTE) <br> * Ansprüche 1-9 * <br> --- | 1-13 | RECHERCHIERTE SACHGEBIETE (Int. Cl.5) |
| P,A | US-A-5 076 983 (LOOMIS ET AL.) <br><br> * Ansprüche 1-15 * <br><br> ----- | 1-13, 18-22,31 | C08L <br> A61L <br> A61K |

Der vorliegende Recherchenbericht wurde für alle Patentansprüche erstellt

| Recherchenort | Abschlußdatum der Recherche | Prüfer |
|---|---|---|
| DEN HAAG | 27 JANUAR 1993 | DECOCKER L. |